# EUROPEAN PATENT SPECIFICATION

(11) **EP 2 702 062 B1**
(45) Date of publication and mention of the grant of the patent: **16.12.2015**
(21) Application number: 12717280.7
(22) Date of filing: 26.04.2012
(51) Int. Cl.: C07D 487/04, A61K 31/4985, A61P 25/18

(54) **SALT OF AN INHIBITOR OF PHOSPHODIESTERASE 10 ENZYME**
SALZ EINES INHIBITORS DES PHOSPHODIESTERASE-10-ENZYMS
SEL D'UN INHIBITEUR DE L'ENZYME PHOSPHODIESTÉRASE 10

(30) Priority: 28.04.2011 US 201113096545
(43) Date of publication of application: 05.03.2014
(73) Proprietor: Janssen Pharmaceutica, N.V., 2340 Beerse (BE)
(72) Inventor: SWINNEY, Kelly Ann, Boston, MA 02127 (US); WUYTS, Stijn, B-2340 Beerse (BE)
(74) Representative: Garcia Prieto, Maria
(86) International application number: PCT/EP2012/057618
(87) International publication number: WO 2012/146644

(56) References cited:
- WO-A1-2004/087710
- WO-A1-2011/051342
- US-A1- 2011 269 752
- KEHLER ET AL.: "The potential therapeutic use of phosphodiesterase 10 inhibitors", EXPERT OPIN. THER. PATENTS, vol. 17, no. 2, 1 February 2007 (2007-02-01), pages 147-158, XP002529529, ISSN: 1354-3776, DOI: 10.1517/13543776.17.12.147

## Description

### Field of the invention

This invention relates to a salt of 3-[6-(2-methoxyethyl)-3-pyridinyl]-2-methyl-8-(4-morpholinyl)-imidazo[1,2-b]pyridazine, a phosphodiesterase 10 enzyme (PDE10) inhibitor, as well as to pharmaceutical compositions comprising the same, the salt for use in the treatment or prevention of a central nervous system disorder, or a metabolic disorder and methods of treatment using the same.

### Background art

The compound 3-[6-(2-methoxyethyl)-3-pyridinyl]-2-methyl-8-(4-morpholinyl)-imidazo[1,2-b]pyridazine, herein referred to as compound 1, has the formula: 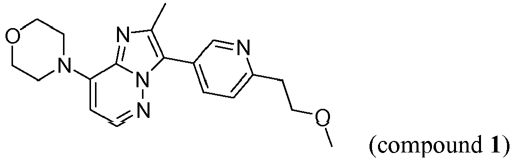 and is described in WO 2011/051342, published on 5-May-2011. The hydrochloride salt (herein referred to as compound **1a**) and the maleate salt (herein referred to as compound **1b**) of 3-[6-(2-methoxyethyl)-3-pyridinyl]-2-methyl-8-(4-morpholinyl)-imidazo[1,2-b]pyridazine as well as the monohydrate (herein referred to as compound **1c**) thereof, are also described in WO 2011/051342.

The compound is an inhibitor of phosphodiesterase 10 enzyme (PDE10) and is a centrally active, potent compound which displays efficacy in preclinical behavior challenge models in which known clinical useful antipsychotics display similar positive responses, such as in the reversal of apomorphine-induced stereotypy and phencyclidine (PCP)-induced hyperlocomotion in rodents. Acute treatment with the compound also reverses the effect of sub-chronic PCP on short-term memory in the novel object recognition (NOR) paradigm, a pre-clinical test modeling a selective deficit in visual episodic memory which is known to be impaired in subjects with schizophrenia. Additionally, the compound reverses the hypolocomotion effects exerted by SCH23390, a D1 receptor antagonist.

A summary of the activity of 3-[6-(2-methoxyethyl)-3-pyridinyl]-2-methyl-8-(4-morpholinyl)-imidazo[1,2-b]pyridazine (compound 1), as disclosed in WO 2011/051342 is displayed in table 1 below:

**Table 1. Pharmacological data for compound 1, as reported in WO 2011/051342. ED₅₀ means effective dose; SD means single dose; DR means dose response; sc means subcutaneous administration; po means per os, oral administration; HPBCD means hydroxypropyl β-cyclodextrin solution.**

| | |
|---|---|
| ***In vitro* PDE10A2 pIC₅₀** | 7.24 |
| **Reversal of apomorphine-induced stereotypy in rodents** | ED₅₀ = 1.3 mg/kg |
| **PDE10 *in vivo* occupancy** | ED₅₀ = 0.48 mg/kg (sc) |
| | ED₅₀ = 1.4 mg/kg (po) |
| | SD % occupancy at 10 mg/kg = 76% (sc) |
| **Reversal of phencyclidine-induced hyperlocomotion in rodents** | ED₅₀ = 2.0 mg/kg |
| **Conditioned avoidance response test** | ED₅₀ = 4.7 mg/kg |
| **Reversal of SCH-23390-induced hypolocomotion in mice** | ED₅₀ = 7.1 mg/kg |
| **Novel object recognition test; retention phase** | 0.3 and 1 mg/kg selectively reversed impairement induced by subchronic PCP. |

Thus, the compound may act as a dopamine modulating agent, inhibiting states of dopaminergic (D₂) hyperactivity and reversing states of dopaminergic (D₁) hypoactivity.

PDE10 inhibitors may possess a pharmacological profile similar to that of the atypical antipsychotics, but lacking the non-target related side effects that are often observed with the currently available antipsychotics; they may further be useful in conditions involving the basal ganglia, such as the conditions described herein, for example, obesity, non-insulin dependent diabetes, bipolar disorder, obsessive compulsive disorder and pain.

### Description of the invention

It has now been found that the phosphate salt of 3-[6-(2-methoxyethyl)-3-pyridinyl]-2-methyl-8-(4-morpholinyl)-imidazo[1,2-b]pyridazine (herein referred to as compound **1d**) constitutes a solid form of robust scalable manufacturability, allowing reproducible stoichiometry control in the salt formation and shows an advantageous balance of physico-chemical properties, such as high solubility, high intrinsic dissolution rate and high resistance to salt dissociation.

Thus, in one aspect, the present invention is directed to the phosphate salt of 3-[6-(2-methoxyethyl)-3-pyridinyl]-2-methyl-8-(4-morpholinyl)-imidazo[1,2-b]pyridazine (compound **1d**).
Said compound **1d** can be represented by the formula:

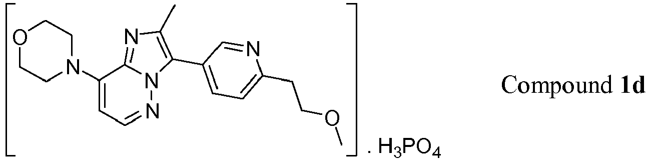

In a further aspect, the invention relates to a pharmaceutical composition comprising:
a therapeutically effective amount of the phosphate salt of 3-[6-(2-methoxyethyl)-3-pyridinyl]-2-methyl-8-(4-morpholinyl)-imidazo[1,2-b]pyridazine (compound **1d**); and
at least one pharmaceutically acceptable carrier, diluent, vehicle or excipient.

In an additional aspect, the invention is also directed to the phosphate salt of 3-[6-(2-methoxyethyl)-3-pyridinyl]-2-methyl-8-(4-morpholinyl)-imidazo[1,2-b]pyridazine (compound **1d**) for use as a medicament, or for use in the treatment or prevention of a central nervous system disorder or of a metabolic disorder.

In a further aspect, the invention relates to the phosphate salt of 3-[6-(2-methoxyethyl)-3-pyridinyl]-2-methyl-8-(4-morpholinyl)-imidazo[1,2-b]pyridazine (compound **1d**) in combination with an additional pharmaceutical agent for use in the treatment or prevention of a central nervous system disorder or of a metabolic disorder.

The invention further relates to a process for preparing a pharmaceutical composition comprising the phosphate salt of 3-[6-(2-methoxyethyl)-3-pyridinyl]-2-methyl-8-(4-morpholinyl)-imidazo[1,2-b]pyridazine (compound **1d**).

In an additional aspect, the invention relates to a product comprising the phosphate salt of 3-[6-(2-methoxyethyl)-3-pyridinyl]-2-methyl-8-(4-morpholinyl)-imidazo [1,2-b]pyridazine (compound **1d**) and an additional pharmaceutical agent, as a combined preparation for simultaneous, separate or sequential use in the treatment or prevention of a central nervous system disorder or of a metabolic disorder.

### Brief description of the drawings

Figure **1a** is an Infrared (IR) spectrum representation of 3-[6-(2-methoxyethyl)-3-pyridinyl]-2-methyl-8-(4-morpholinyl)-imidazo[1,2-b]pyridazine (compound 1).
Figure **1b** is an Infrared (IR) spectrum representation of 3-[6-(2-methoxyethyl)-3-pyridinyl]-2-methyl-8-(4-morpholinyl)-imidazo[1,2-b]pyridazine monophosphate salt (compound **1d**).
Figure **2a** is a powder X-Ray Diffraction (XRD) pattern representation of 3-[6-(2-methoxyethyl)-3-pyridinyl]-2-methyl-8-(4-morpholinyl)-imidazo[1,2-b]pyridazine (compound **1**).
Figure **2b** is a powder X-Ray Diffraction (XRD) pattern representation of 3-[6-(2-methoxyethyl)-3-pyridinyl]-2-methyl-8-(4-morpholinyl)-imidazo[1,2-b]pyridazine monophosphate (compound **1d**).

### Detailed description of the invention

The present invention is directed to the phosphate salt of 3-[6-(2-methoxyethyl)-3-pyridinyl]-2-methyl-8-(4-morpholinyl)-imidazo[1,2-b]pyridazine (compound **1d**). Said phosphate salt corresponds to the monophosphate salt of 3-[6-(2-methoxyethyl)-3-pyridinyl]-2-methyl-8-(4-morpholinyl)-imidazo[1,2-b]pyridazine.

The phosphate salt of 3-[6-(2-methoxyethyl)-3-pyridinyl]-2-methyl-8-(4-morpholinyl)-imidazo[1,2-b]pyridazine (compound **1d**) can be prepared by reacting the corresponding free base (compound **1**) with phosphoric acid (H₃PO₄) in the presence of a suitable solvent, such as for example, an alcohol or a mixture of two or more alcohols. Typical alcohols are selected from but not limited to methanol, ethanol, propanol, isopropanol, butanol, sec-butanol, tert-butanol, or a mixture thereof. Mixtures of alcohols are selected from, but not limited to ethanol/butanol.

The PDE10 inhibiting activity of 3-[6-(2-methoxyethyl)-3-pyridinyl]-2-methyl-8-(4-morpholinyl)-imidazo[1,2-b]pyridazine (compound **1**) and synthesis thereof, are described in WO 2011/051342, which is incorporated herein by reference.

The phosphate salt of 3-[6-(2-methoxyethyl)-3-pyridinyl]-2-methyl-8-(4-morpholinyl)-imidazo[1,2-b]pyridazine (compound **1d**) has been found to display a beneficial balance of properties. Compound **1d** has a higher solubility than the corresponding 3-[6-(2-methoxyethyl)-3-pyridinyl]-2-methyl-8-(4-morpholinyl)-imidazo[1,2-b] pyridazine in anhydrous free base form (compound **1**), and displays a higher intrinsic dissolution rate compared to that of both, the anhydrous free base and the monohydrate of 3-[6-(2-methoxyethyl)-3-pyridinyl]-2-methyl-8-(4-morpholinyl)-imidazo[1,2-b] pyridazine (compounds **1** and **1c**, respectively). Additionally, compound **1d** constitutes a solid form of robust scalable manufacturability, allowing reproducible stoichiometry control in the salt formation. Finally, the melting point measured for compound **1d** (DSC, mp = 165.1°C) is higher than that measured for the maleate salt of 3-[6-(2-methoxyethyl)-3-pyridinyl]-2-methyl-8-(4-morpholinyl)-imidazo[1,2-b]pyridazine (compound **1b**) (DSC, mp = 113.8 °C) which may provide higher resistance to potential salt dissociation phenomena upon mechanical manipulation and formulation during production and/or upon storage of the formulated product.

### Pharmacology

Compounds that inhibit PDE10 enzyme activity, in particular PDE10A enzyme activity, raise the levels of cAMP or cGMP within cells that express PDE10. Accordingly, inhibition of PDE10 enzyme activity may be useful in the treatment of diseases caused by deficient amounts of cAMP or cGMP in cells. PDE10 inhibitors may also be of benefit in cases in which raising the amount of cAMP or cGMP above normal levels results in a therapeutic effect. Inhibitors of PDE10 may be used to treat disorders of the peripheral and central nervous system, cardiovascular diseases, cancer, gastro-enterological diseases, endocrinological or metabolic diseases and urological diseases.

Hence, the present invention relates to the phosphate salt of 3-[6-(2-methoxyethyl)-3-pyridinyl]-2-methyl-8-(4-morpholinyl)-imidazo[1,2-b]pyridazine (compound **1d**) according to the present invention for use as a medicine, as well as to the use of compound **1d** according to the invention or a pharmaceutical composition according to the invention for the manufacture of a medicament. The present invention also relates to compound **1d** according to the present invention or a pharmaceutical composition according to the invention for use in the treatment or prevention of, in particular treatment of, a condition in a mammal, including a human, the treatment or prevention of which is affected or facilitated by the inhibition of phosphodiesterase 10 enzyme. The present invention also relates to the use of compound **1d** according to the present invention or a pharmaceutical composition according to the invention for the manufacture of a medicament for the treatment or prevention of, in particular treatment of, a condition in a mammal, including a human, the treatment or prevention of which is affected or facilitated by the inhibition of phosphodiesterase 10 enzyme.

The present invention also relates to compound **1d** according to the invention, or a pharmaceutical composition according to the invention for use in the treatment, prevention, amelioration, control or reduction of the risk of various neurological, psychiatric and metabolic disorders associated with phosphodiesterase 10 dysfunction in a mammal, including a human, the treatment or prevention of which is affected or facilitated by the inhibition of phosphodiesterase 10.

Also, the present invention relates to the use of compound **1d** according to the invention or a pharmaceutical composition according to the invention for the manufacture of a medicament for treating, preventing, ameliorating, controlling or reducing the risk of various neurological and psychiatric disorders associated with phosphodiesterase 10 dysfunction in a mammal, including a human, the treatment or prevention of which is affected or facilitated by the inhibition of phosphodiesterase 10.

Where the invention is said to relate to the use of compound **1d** or composition according to the invention for the manufacture of a medicament for e.g. the treatment of a mammal, it is understood that such use is to be interpreted in certain jurisdictions as a method of e.g. treatment of a mammal, comprising administering to a mammal in need of such e.g. treatment, an effective amount of compound **1d** or composition according to the invention.

In particular, the indications that may be treated with PDE10 inhibitors, either alone or in combination with other drugs, include, but are not limited to, those diseases thought to be mediated in part by the basal ganglia, prefrontal cortex and hippocampus.

These indications include neurological and psychiatric disorders selected from psychotic disorders and conditions; anxiety disorders; movement disorders; drug abuse; mood disorders; neurodegenerative disorders; disorders or conditions comprising as a symptom a deficiency in attention and/or cognition; pain and metabolic disorders.

In particular, the psychotic disorders and conditions associated with PDE10 dysfunction include one or more of the following conditions or diseases: schizophrenia, for example of the paranoid, disorganized, catatonic, undifferentiated or residual type; schizophreniform disorder; schizoaffective disorder, such as delusional or depressive type; delusional disorder; substance-induced psychotic disorder such as psychosis induced by alcohol, amphetamine, cannabis, cocaine, hallucinogens, inhalants, opioids, or phencyclidine; personality disorders of the paranoid type; and personality disorder of the schizoid type.

In particular, the anxiety disorders include panic disorder; agoraphobia; specific phobia; social phobia; obsessive-compulsive disorder; post-traumatic stress disorder; acute stress disorder; and generalized anxiety disorder.

In particular, movement disorders include Huntington's disease and dyskinesia; Parkinson's disease; restless leg syndrome and essential tremor. Additionally, Tourette's syndrome and other tic disorders can be included.

In particular, the central nervous system disorder is a substance-related disorder selected from the group of alcohol abuse; alcohol dependence; alcohol withdrawal; alcohol withdrawal delirium; alcohol-induced psychotic disorder; amphetamine dependence; amphetamine withdrawal; cocaine dependence; cocaine withdrawal; nicotine dependence; nicotine withdrawal; opioid dependence and opioid withdrawal.

In particular, mood disorders and mood episodes include depression, mania and bipolar disorders. Preferably, the mood disorder is selected from the group of bipolar disorders (I and II); cyclothymic disorder; depression; dysthymic disorder; major depressive disorder and substance-induced mood disorder.

In particular, neurodegenerative disorders include Parkinson's disease; Huntington's disease; dementia such as for example Alzheimer's disease; multi-infarct dementia; AIDS-related dementia or fronto temperal dementia. The neurodegenerative disorder or condition comprises neurodegeneration of striatal medium spiny neurons.

In particular, disorders or conditions comprising as a symptom a deficiency in attention and/or cognition include dementia, such as Alzheimer's disease; multi-infarct dementia; alcoholic dementia or drug-related dementia; dementia associated with intracranial tumours or cerebral trauma; dementia associated with Huntington's disease; dementia associated with Parkinson's disease; AIDS-related dementia; other diseases include delirium; amnestic disorder; post-traumatic stress disorder; mental retardation; a learning disorder; attention-deficit/hyperactivity disorder (ADHD); and age-related cognitive impairment.

In particular, pain includes acute and chronic states, severe pain, intractable pain, neuropathic pain and post-traumatic pain.

In particular, metabolic disorders include diabetes, in particular type 1 or type 2 diabetes, and related disorders such as obesity. Additional related disorders include syndrome X, impaired glucose tolerance, impaired fasting glucose, gestational diabetes, maturity-onset diabetes of the young (MODY), latent autoimmune diabetes adult (LADA), associated diabetic dyslipidemia, hyperglycemia, hyperinsulinemia, dyslipidemia, hypertriglyceridemia, and insulin resistance.

Additionally, the growth of some cancer cells is inhibited by cAMP and cGMP, thus compound **1d** may be useful in the treatment of cancer, such as renal carcinoma and breast cancer.

Preferably, the psychotic disorder is selected from the group of schizophrenia, delusional disorder, schizoaffective disorder, schizophreniform disorder and substance-induced psychotic disorder.

Preferably, the central nervous system disorder is a personality disorder selected from the group of obsessive-compulsive personality disorder and schizoid, schizotypal disorder.

Preferably, the central nervous system disorder is a mood disorder selected from the group of bipolar disorders (I & II), cyclothymic disorder, depression, dysthymic disorder, major depressive disorder and substance-induced mood disorder.

Preferably, the central nervous system disorder is attention-deficit/hyperactivity disorder.

Preferably, the central nervous system disorder is a cognitive disorder selected from the group of delirium, substance-induced persisting delirium, dementia, dementia due to HIV disease, dementia due to Huntington's disease, dementia due to Parkinson's disease, dementia of the Alzheimer's type, substance-induced persisting dementia and mild cognitive impairment.

Preferably the disorders treated by compound **1d** of the present invention are selected from schizophrenia; obsessive-compulsive disorder; generalized anxiety disorder; Huntington's disease; dyskinesia; Parkinson's disease; depression; bipolar disorders; dementia such as Alzheimer's disease; attention-deficit/hyperactivity disorder; drug abuse; pain; diabetes and obesity.

Preferably, the disorders treated by compound **1d** of the present invention are schizophrenia, including positive and negative symptoms thereof, and cognitive deficits, such as impaired attention or memory.

Of the disorders mentioned above, the treatment of anxiety, obsessive-compulsive disorder, schizophrenia, depression, attention-deficit/hyperactivity disorder, Alzheimer's disease and diabetes are of particular importance.

At present, the fourth edition of the Diagnostic & Statistical Manual of Mental Disorders (DSM-IV) of the American Psychiatric Association provides a diagnostic tool for the identification of the disorders described herein. The person skilled in the art will recognize that alternative nomenclatures, nosologies, and classification systems for neurological and psychiatric disorders described herein exist, and that these evolve with medical and scientific progresses.

Therefore, the invention also relates to the phosphate salt of 3-[6-(2-methoxyethyl)-3-pyridinyl]-2-methyl-8-(4-morpholinyl)-imidazo[1,2-b]pyridazine (compound **1d**) according to the invention, for use in the treatment of any one of the diseases mentioned hereinbefore.

The invention also relates to compound **1d** according to the invention for use in treating any one of the diseases mentioned hereinbefore.

The invention also relates to compound **1d** according to the invention, for the treatment or prevention, in particular treatment, of any one of the diseases mentioned hereinbefore.

The invention also relates to the use of compound **1d** according to the invention, for the manufacture of a medicament for the treatment or prevention of any one of the disease conditions mentioned hereinbefore.

The invention also relates to the use of compound **1d** according to the invention for the manufacture of a medicament for the treatment of any one of the disease conditions mentioned hereinbefore.

Compound **1d** of the present invention can be administered to mammals, preferably humans, for the treatment or prevention of any one of the diseases mentioned hereinbefore.

In view of the utility of compound **1d** according to the invention, there is provided a method of treating warm-blooded animals, including humans, suffering from any one of the diseases mentioned hereinbefore, and a method of preventing in warm-blooded animals, including humans, any one of the diseases mentioned hereinbefore.

Said methods comprise the administration, i.e. the systemic or topical administration, preferably oral administration, of a therapeutically effective amount of compound **1d** according to the invention to warm-blooded animals, including humans.

Therefore, the invention also relates to a method for the prevention and/or treatment of any one of the diseases mentioned hereinbefore comprising administering a therapeutically effective amount of compound **1d** according to the invention to a patient in need thereof.

Compound **1d**, described herein, can be used alone or in combination with other pharmaceutical agents such as other agents used in the treatment of psychoses, such as schizophrenia and bipolar disorder, obsessive-compulsive disorder, Parkinson's disease, cognitive impairment and/or memory loss, e.g. nicotinic α-7 agonists, PDE4 inhibitors, other PDE10 inhibitors, calcium channel blockers, muscarinic m1 and m2 modulators, adenosine receptor modulators, ampakines, NMDA-R modulators, mGluR modulators, dopamine modulators, serotonin modulators, cannabinoid modulators, and cholinesterase inhibitors (e.g. donepezil, rivastigmine, and galantamine). In such combinations, compound **1d** of the present invention may be utilized in combination with one or more other drugs in the treatment, prevention, control, amelioration, or reduction of risk of diseases or conditions for which compound **1d** or the other drugs may have utility, where the combination of the drugs together are safer or more effective than either drug alone.

One skilled in the art will recognize that a therapeutically effective amount of compound **1d** of the present invention is the amount sufficient to inhibit the PDE10 enzyme and that this amount varies *inter alia*, depending on the type of disease, the concentration of compound **1d** in the therapeutic formulation, and the condition of the patient. Generally, an amount of compound **1d** to be administered as a therapeutic agent for treating diseases in which inhibition of the PDE10 enzyme is beneficial, such as the disorders described herein, will be determined on a case by case by an attending physician.

Generally, a suitable dose is one that results in a concentration of the PDE10 inhibitor at the treatment site in the range of 0.5 nM to 200 µM, and more usually 5 nM to 50 µM. To obtain these treatment concentrations, a patient in need of treatment likely will be administered between 0.001 mg/kg to 15 mg/kg body weight, in particular from 0.01 mg/kg to 2.50 mg/kg body weight, in particular, from 0.01 to 1.5 mg/kg body weight, in particular from 0.1 mg/kg to 0.50 mg/kg body weight. The amount of compound **1d** according to the present invention, also referred to here as the active ingredient, which is required to achieve a therapeutical effect will, of course vary on case-by-case basis, vary with the route of administration, the age and condition of the recipient, and the particular disorder or disease being treated. A method of treatment may also include administering the active ingredient on a regimen of between one and four intakes per day. In these methods of treatment compound **1d** according to the invention is preferably formulated prior to admission. As described herein below, suitable pharmaceutical formulations are prepared by known procedures using well known and readily available ingredients.

### Pharmaceutical compositions

The present invention also provides compositions for preventing or treating diseases in which inhibition of the PDE10 enzyme is beneficial, such as the disorders described herein. While it is possible for the active ingredient to be administered alone, it is preferable to present it as a pharmaceutical composition. Accordingly, the present invention also relates to a pharmaceutical composition comprising a pharmaceutically acceptable carrier or diluent and, as active ingredient, a therapeutically effective amount of compound **1d** according to the invention. The carrier or diluent must be "acceptable" in the sense of being compatible with the other ingredients of the composition and not deleterious to the recipients thereof.

Compound **1d** according to the invention, may be formulated into various pharmaceutical forms for administration purposes. As appropriate compositions there may be cited all compositions usually employed for systemically administering drugs.

The pharmaceutical compositions of this invention may be prepared by any methods well known in the art of pharmacy, for example, using methods such as those described in Gennaro et al. Remington's Pharmaceutical Sciences (18th ed., Mack Publishing Company, 1990, see especially Part 8: Pharmaceutical preparations and their Manufacture). To prepare the pharmaceutical compositions of this invention, a therapeutically effective amount of compound **1d** as the active ingredient is combined in intimate admixture with a pharmaceutically acceptable carrier or diluent, which carrier or diluent may take a wide variety of forms depending on the form of preparation desired for administration. These pharmaceutical compositions are desirable in unitary dosage form suitable, in particular, for oral, topical (for example via a nose spray, eye drops or via a cream, gel, shampoo or the like), rectal or percutaneous administration, by parenteral injection or by inhalation, such as a nose spray. For example, in preparing the compositions in oral dosage form, any of the usual pharmaceutical media may be employed such as, for example, water, glycols, oils, alcohols and the like in the case of oral liquid preparations such as, for example, suspensions, syrups, elixirs, emulsions and solutions; or solid carriers such as, for example, starches, sugars, kaolin, diluents, lubricants, binders, disintegrating agents and the like in the case of powders, pills, capsules and tablets. Because of the ease in administration, oral administration is preferred, and tablets and capsules represent the most advantageous oral dosage unit forms in which case solid pharmaceutical carriers are employed. For parenteral compositions, the carrier will usually comprise sterile water, at least in large part, though other ingredients, for example, surfactants to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations that are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, said additives do not introduce a significant deleterious effect on the skin. Said additives may facilitate the administration to the skin and/or may be helpful for preparing the desired compositions. These compositions may be administered in various ways, e.g., as a transdermal patch, as a spot-on treatment, as an ointment.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, teaspoonfuls, tablespoonfuls, and segregated multiples thereof.

Since compound **1d** according to the invention is orally administrable, pharmaceutical compositions comprising aid compounds for oral administration are especially advantageous.

In order to enhance the solubility and/or the stability of compound **1d** in pharmaceutical compositions, it can be advantageous to employ α-, β- or γ-cyclodextrins or their derivatives, in particular hydroxyalkyl substituted cyclodextrins, e.g. 2-hydroxypropyl-p-cyclodextrin or sulfobutyl-β-cyclodextrin. Also co-solvents such as alcohols may improve the solubility and/or the stability of compound **1d** according to the invention in pharmaceutical compositions.

The exact dosage and frequency of administration of compound **1d** used, depends on the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing compound **1d** of the instant invention.

Depending on the mode of administration, the pharmaceutical composition will comprise from 0.05 to 99 % by weight, preferably from 0.1 to 70 % by weight, more preferably from 0.1 to 50 % by weight of the active ingredient, and, from 1 to 99.95 % by weight, preferably from 30 to 99.9 % by weight, more preferably from 50 to 99.9 % by weight of a pharmaceutically acceptable carrier, all percentages being based on the total weight of the composition.

The amount of compound **1d** that can be combined with a carrier material to produce a single dosage form will vary depending upon the disease treated, the mammalian species, and the particular mode of administration. However, as a general guide, suitable unit doses for compound **1d** of the present invention can, for example, preferably contain between 0.1 mg to about 1000 mg of the active compound **1d**. A preferred unit dose is between 1 mg to about 500 mg. A more preferred unit dose is between 1 mg to about 300 mg. Even more preferred unit dose is between 1 mg to about 100 mg. Such unit doses can be administered more than once a day, for example, 2, 3, 4, 5 or 6 times a day, but preferably 1 or 2 times per day, so that the total dosage for a 70 kg adult is in the range of 0.001 to about 15 mg per kg weight of subject per administration. A preferred dosage is 0.01 to about 1.5 mg per kg weight of subject per administration, and such therapy can extend for a number of weeks or months, and in some cases, years. It will be understood, however, that the specific dose level for any particular patient will depend on a variety of factors including the activity of the specific compound employed; the age, body weight, general health, sex and diet of the individual being treated; the time and route of administration; the rate of excretion; other drugs that have previously been administered; and the severity of the particular disease undergoing therapy, as is well understood by those of skill in the area.

A typical dosage can be one 1 mg to about 100 mg tablet or 1 mg to about 300 mg taken once a day, or, multiple times per day, or one time-release capsule or tablet taken once a day and containing a proportionally higher content of active ingredient. The time-release effect can be obtained by capsule materials that dissolve at different pH values, by capsules that release slowly by osmotic pressure, or by any other known means of controlled release.

It can be necessary to use dosages outside these ranges in some cases as will be apparent to those skilled in the art. Further, it is noted that the clinician or treating physician will know how and when to start, interrupt, adjust, or terminate therapy in conjunction with individual patient response.

As already mentioned, the invention also relates to a pharmaceutical composition comprising compound **1d** according to the invention and one or more other drugs for use as a medicament or for use in the treatment, prevention, control, amelioration, or reduction of risk of diseases or conditions for which compound **1d** or the other drugs may have utility as well. The use of such a composition for the manufacture of a medicament, as well as the use of such a composition for the manufacture of a medicament in the treatment, prevention, control, amelioration or reduction of risk of diseases or conditions for which compound **1d** or the other drugs may have utility are also contemplated. The present invention also relates to a combination of compound **1d** according to the present invention and an additional pharmaceutical agent. The present invention also relates to such a combination for use as a medicine. The present invention also relates to a product comprising (a) compound **1d** according to the present invention, and (b) an additional pharmaceutical agent, as a combined preparation for simultaneous, separate or sequential use in the treatment or prevention of a condition in a mammal, including a human, the treatment or prevention of which is affected or facilitated by the effect of PDE10 inhibitors, in particular PDE10A inhibitors. The different drugs of such a combination or product may be combined in a single preparation together with pharmaceutically acceptable carriers or diluents, or they may each be present in a separate preparation together with pharmaceutically acceptable carriers or diluents.

The following examples are intended to illustrate but not to limit the scope of the present invention. In the examples below, the indicated melting points for compound 1 and the different salts and solvate forms thereof were recorded on a TA-Instruments Q1000 MTDSC equipped with a RCS cooling unit using the parameters indicated hereinbelow.

### Example 1. Preparation of salt and solvate forms of 3-[6-(2-methoxyethyl)-3-pyridinyl]-2-methyl-8-(4-morpholinyl)-imidazo[1,2-b]pyridazine (compound 1)

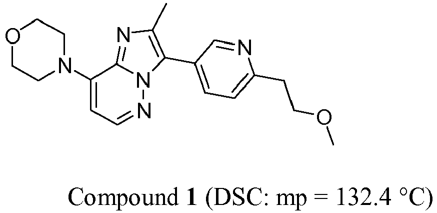

### Formation of the hydrochloride salt of 3-[6-(2-methoxyethyl)-3-pyridinyl]-2-methyl-8-(4-morpholinyl)-imidazo[1,2-b]pyridazine (compound 1a):

To a stirred solution of 3-[6-(2-methoxyethyl)-3-pyridinyl]-2-methyl-8-(4-morpholinyl)-imidazo[1,2-b]pyridazine (prepared according to the procedure described in
WO 2011/051342) (0.5 g, 1.41 mmol) in 2-butanone (7.50 mL) was added hydrogen chloride (1 N, 1.27 mL, 1.27 mmol). The mixture was concentrated in vacuo till dry at 50 °C, then 2-butanone (7.50 mL) was added. Formation of a white solid was observed and the suspension was further stirred at room temperature overnight. The solid was filtered off and dried overnight at 50 °C to yield compound **1a** (0.397 g, 72%) as a white solid.

Hydrochloride salt (.HCl) (DSC: mp = 164.8 °C with decomposition.

### Formation of the maleate salt of 3-[6-(2-methoxyethyl)-3-pyridinyl]-2-methyl-8-(4-morpholinyl)-imidazo[1,2-b]pyridazine (compound 1b):

To a mixture of 3-[6-(2-methoxyethyl)-3-pyridinyl]-2-methyl-8-(4-morpholinyl)-imidazo[1,2-b]pyridazine (prepared according to the procedure described in WO 2011/051342) (1.00 g, 2.83 mmol) and (Z)-2-butenedioic acid (344.85 mg, 2.97 mmol), was added 1-methoxy-2-propanol (15.00 mL) and the mixture was heated to 40 °C then cooled down to 0 °C and stirred overnight at 0 °C. Precipitation was observed, with formation of very fine solids, giving the reaction mixture an almost milky appearance. A small amount of these solids was set aside and used subsequently as seeding material (see below). The mixture was heated to 50 °C. The solvent was partially evaporated to a volume of 10 mL and the reaction mixture was seeded with original solids at 45 °C. Stirring was continued at 45 °C for 4 hours, then at 40 °C for 2 hours and continued at
20 °C overnight. The resulting solid was filtered off, washed once with a very small amount of propylene glycol monomethyl ether (PGME) and once with methyl tert-butyl ether (MTBE) and dried overnight at 45 °C, to yield compound **1b** (850 mg, 64%) as a white to slightly yellow solid.

Maleate salt (.C₄H₄O₄) (DSC: mp = 113.8 °C).

### Formation of the hydrate of 3-[6-(2-methoxyethyl)-3-pyridinyl]-2-methyl-8-(4-morpholinyl)-imidazo[1,2-b]pyridazine (compound 1c):

For the preparation of the hydrate, two different procedures were used:
a) water (2 mL) was added to the solid 3-[6-(2-methoxyethyl)-3-pyridinyl]-2-methyl-8-(4-morpholinyl)-imidazo[1,2-b]pyridazine (prepared according to the procedure described in WO 2011/051342) (20 mg) and allowed to slurry at 70 °C for 30 minutes. The heat was turned off and the solution mixture was allowed to continue to slurry at room temperature for 3 days.
b) water (4 mL) was added to the solid 3-[6-(2-methoxyethyl)-3-pyridinyl]-2-methyl-8-(4-morpholinyl)-imidazo[1,2-b]pyridazine (prepared according to the procedure described in WO 2011/051342) (200 mg) and allowed to slurry at room temperature for 5 days.

The product was then filtered off and dried to yield the hydrate as a solid. Monohydrate (.H₂O) (DSC: dehydration between about 55-90 °C followed by melt at 132.9 °C).

### Formation of the monophosphate salt of 3-[6-(2-methoxyethyl)-3-pyridinyl]-2-methyl-8-(4-morpholinyl)-imidazo[1,2-b]pyridazine (compound 1d):

A stirred mixture of 3-[6-(2-methoxyethyl)-3-pyridinyl]-2-methyl-8-(4-morpholinyl)-imidazo[1,2-b]pyridazine (prepared according to the procedure described in WO 2011/051342) (1g, 2.83 mmol), phosphoric acid (0.342 g, aqueous solution 85 wt%) and a small volume of a 50/50 v/v ethanol:1-butanol mixture (2 mL) was heated to reflux. An additional amount of the 50/50 ethanol:1-butanol solvent mixture was added portion-wise (up to a total of 14.65 g of the solvent mixture) to the refluxed reaction mixture, until a clear solution was obtained. The resulting solution was cooled from reflux temperature to room temperature over a period of 30 minutes. Formation of a solid was observed, which was filtered to result in 5 g of a wet material that was dried under vacuum for about 2 hours at 50 °C, for 72 hours at room temperature, and for 4 hours at 50 °C to yield compound **1d** as a crystalline solid (1 g, 78 %).
Phosphate salt (.H₃PO₄) (DSC: mp = 165.1 °C with decomposition)

### Example 2. Characterization of compound 1 (free base form) and the phosphate salt of compound 1 (compound 1d)

The results of the characterization by infrared spectrometry (IR), powder X-ray diffraction (XRD), differential scanning calorimetry (DSC), thermogravimetric analysis (TGA) and dynamic vapor sorption (DVS) characteristics for compound **1** (free base form) and the phosphate salt of compound **1** (compound **1d**) are described below.

### Micro Attenuated Total Reflectance (microATR)

Each sample was analyzed using a suitable microATR accessory as described below.

| | |
|---|---|
| number of scans: | 32 |
| resolution: | 1 cm-1 |
| wavelength range: | 4000 to 400 cm-1 |
| apparatus: | Thermo Nexus 670 FTIR spectrometer |
| baseline correction: | yes |
| detector: | DTGS with KBr windows |
| beamsplitter: | Ge on KBr |
| micro ATR accessory: | Harrick Split Pea with Si crystal |

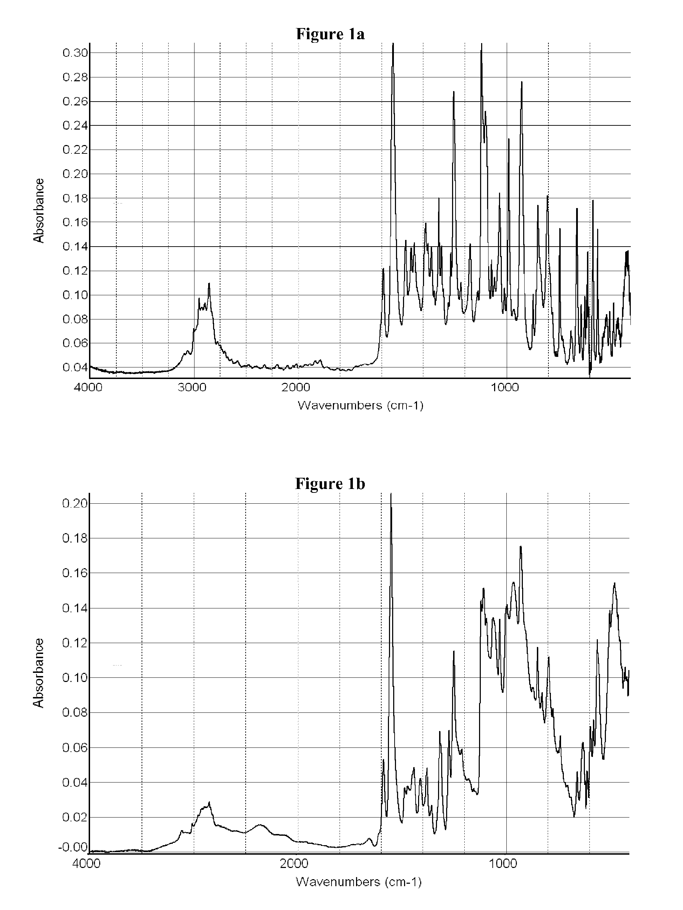

The IR spectrum of compound **1** (free base form), is as substantially depicted in Figure 1a, and reflects the vibrational modes of the molecular structure of the compound. Compound **1** is characterized by an FTIR spectrum with typical absorption bands at 1591,1545, 1483, 1458, 1443, 1389, 1377, 1361, 1325, 1311, 1268, 1254, 1175, 1119, 1102, 1071, 1033, 990, 928, 849, 804, 745, 661, 585 and 563 cm⁻¹ ±2 cm^{-1.}.

The IR spectrum of compound **1d** (the phosphate salt of compound 1) is as substantially depicted in Figure 1b, and contains no bands corresponding to the free base. The spectrum reflects the vibrational modes of the molecular structure of the compound and shows the presence of characteristic bands for phosphate salt. Compound **1d** is characterized by an FTIR spectrum with typical absorption bands at about 1589, 1552, 1318, 1275, 1250, 1120, 1108, 1095, 1064, 1031, 996, 964, 931, 849, 827, 795, 740, 595, 581, 563, 502 and 478 cm⁻¹ ±2 cm⁻¹.

### Powder XRD

X-ray powder diffraction (XRPD) analysis was carried out on a Philips X'PertPRO MPD diffractometer PW3050/60 with generator PW3040. The instrument was equipped with a Cu LFF X-ray tube PW3373/10. The compound was spread on a zero background sample holder.

### INSTRUMENT PARAMETERS

| | |
|---|---|
| generator voltage: | 45 kV |
| generator amperage: | 40 mA |
| geometry: | Bragg-Brentano |
| stage: | spinner stage |

### MEASUREMENT CONDITIONS

| | |
|---|---|
| scan mode: | continuous |
| scan range: | 3 to 50° 2θ |
| step size: | 0.0167°/step |
| counting time: | 29.845 sec/step |
| spinner revolution time: | 1 sec |
| radiation type: | CuKα |

### Incident beam path Diffracted beam path

| | | | |
|---|---|---|---|
| program. divergence slit: | 15 mm | long anti scatter shield: | + |
| Soller slit: | 0.04 rad | Soller slit: | 0.04 rad |
| beam mask: | 15 mm | Ni filter: | + |
| anti scatter slit: | 1° | detector: | X'Celerator |
| beam knife: | + | | |

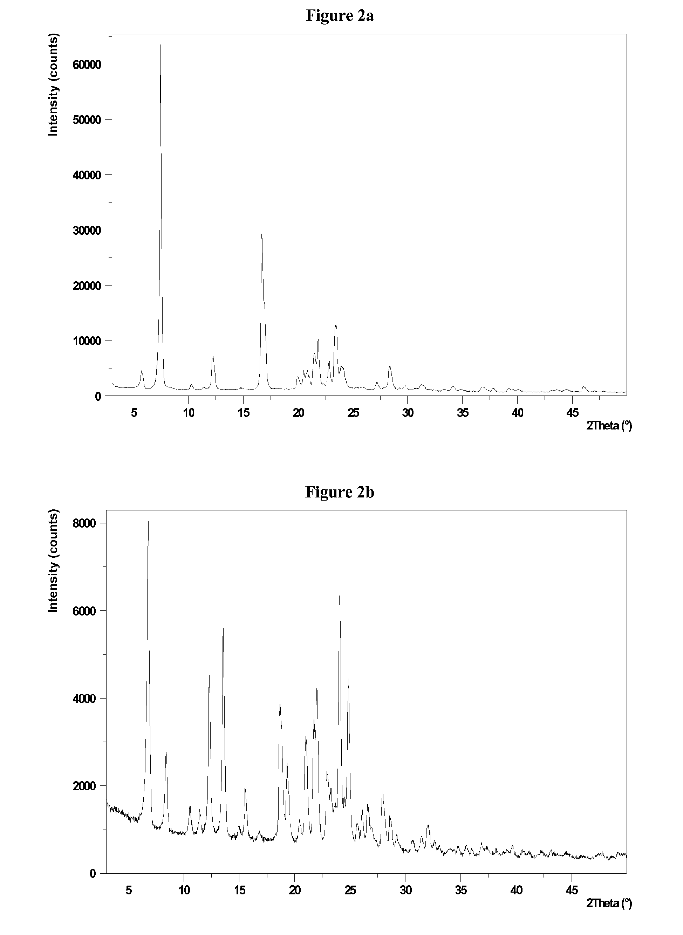

The X-ray powder diffraction pattern of compound **1** is as substantially depicted in Figure 2a and shows diffraction peaks without the presence of a halo, indicating that this compound is present as a crystalline product.
The X-ray powder diffraction pattern of compound **1d** (the phosphate salt of compound 1) is as substantially depicted in Figure 2b and shows diffraction peaks without the presence of a halo, indicating that this compound is present as a crystalline product, containing no free base.

### Differential scanning calorimetry (DSC)

A sample of about 3 mg of the compound was transferred into a standard aluminum TA-Instrument sample pan. The sample pan was closed with the appropriate cover and the DSC curve was recorded on a TA-Instruments Q1000 MTDSC equipped with a RCS cooling unit using the following parameters:

| | |
|---|---|
| initial temperature: | 25°C |
| heating rate: | 10°C/min |
| final temperature: | 300°C |
| nitrogen flow: | 50 ml/min |

It was found that compound **1** melts at 132.4°C with a heat of fusion is 96 J/g and that compound **1d** (the phosphate salt of compound 1) melts with decomposition at about 165.1°C.

### Thermogravimetry (TGA)

A sample of the compound was transferred into an aluminum sample pan. The TG curve was recorded on a TA Instruments Q500 thermogravimeter using the following parameters:

| | |
|---|---|
| initial temperature: | room temperature |
| heating rate: | 20°C/min |
| resolution factor: | 4 |
| final condition: | 300°C or <80[(w/w)%] |

For compound **1**, a weight loss of 0.17% in the temperature region from room temperature up to 125°C and of 0.16% between 125 and 190°C were recorded. Additional weight loss was observed above 190 °C.

For compound **1d** (the phosphate salt of compound **1**), a weight loss of 0.29% in the temperature region from room temperature up to 150°C and a weight loss of 7.5% between 150 and 200°C were observed.

### Adsorption-Desorption

A sample of compound (about 7 mg of compound **1** and about 12 mg in the case of compound **1d** (the phosphate salt of compound **1**)) was transferred into a SMS dynamic vapor sorption model DVS-Advantage and the weight change with respect to the atmospheric humidity at 25°C was recorded using the following parameters (RH means relative humidity):

| | |
|---|---|
| drying: | 60 min. under dry nitrogen at 25°C |
| equilibrium: | ≤0.01%/min. for min:15 min and max: 60min. |

| RH (%) measurement points: | |
|---|---|
| first set: | 5,10,20,30,40,50,60,70,80,90,95,90,80,70,60,50,40,30,20,10,5 |
| second set: | 5,10,20,30,40,50,60,70,80,90,95,90,80,70,60,50,40,30,20,10,5,0 |

During the initial drying step, a weight loss of 0.06% was registered for compound **1**. The product showed no hygroscopic behavior and remained the same physical form during the test.
During the initial drying step, a weight loss of 0.3% was registered for compound **1d** (the phosphate salt of compound **1**). The product shows no hygroscopic behavior and remains crystalline during the test. No dissociation of the salt was observed.

### Solubility of crystalline form of compound 1 (anhydrous free base) and the phosphate salt of compound 1 (compound 1d):

An excess of compound **1** (anhydrous free base) or of compound **1d** (phosphate salt of compound 1), respectively was equilibrated with the solvent at 20 °C for at least 24 hours. After removing the undissolved compound (0.45 µm Millex LCR filter), the concentration in the solution was determined using UV spectrometry. The pH of the solution was measured. The results are shown in tables 2a and 2b below.

**Table 2a. Solubility as a function of pH at 20 °C of compound 1 (anhydrous free base)**

| **Solvent** | **Solubility (mg/mL) of compound 1** | **pH of solution** |
|---|---|---|
| **water** | 0.009 | 8.7 |
| **0.1N HCl** | 2.5 | 2.5 |
| **0.01N HCl** | 0.32 | 3.0 |
| **citrate**-**NaOH**-**HCl buffer pH 2** | 0.61 | 2.9 |
| **citrate-NaOH buffer pH 5** | 0.012 | 5.0 |
| **phosphate buffer pH** 7 | 0.008 | 7.0 |
| **borate**-**KCl**-**NaOH buffer pH 9** | 0.009 | 8.9 |
| **phosphate**-**NaOH buffer pH 12** | 0.008 | 11.9 |
| **0**.**1N NaOH** | 0.008 | 12.9 |

**Table 2b. Solubility as a function of pH at 20 °C of compound 1d (phosphate salt of compound 1)**

| **Solvent** | **Solubility (mg/mL) of compound 1d** | **pH of solution** |
|---|---|---|
| **water** | 9.7 | 2.6 |
| **0.1N HCl** | 46.0 | 2.1 |
| **0**.**01N HCl** | 11.9 | 2.5 |
| **citrate**-**NaOH**-**HCl buffer pH 2** | 20.2 | 2.5 |
| **citrate-NaOH buffer pH 5** | 0.14 | 4.9 |
| **phosphate buffer pH 7** | 0.09 | 6.8 |
| **borate**-**KCl**-**NaOH buffer pH 9** | 0.10 | 7.4 |
| **phosphate**-**NaOH buffer pH 12** | 0.10 | 11.2 |
| **0**.**1N NaOH** | 0.10 | 12.5 |

### Intrinsic Dissolution Rate (IDR) of compound 1 (anhydrous free base), compound 1d (phosphate salt of compound 1) and compound 1c (monohydrate form of compound 1) pressed as miniaturized pellets

Dissolution rate measurements were performed using ±5 mg of compound **1** (anhydrous free base), compound **1d** (phosphate salt) or compound **1c** (monohydrate form), respectively pressed as pellets (of 0.0754 cm² area) made in a Mini-IDR^{™} compression system (from pION) with a compression of 40 bar for 1 min. The intrinsic dissolution rate was measured using a DISS Profiler^{™} system consisting in both a mini-bath and UV fiber optic (5 mm pathlength) probes connected to a photodiode array (PDA) spectrophotometer (from pION Inc). Each pellet prepared were positioned in individual vessels. The intrinsic dissolution test was performed using 0.01 M HCl (20 mL) maintained at 37.0 ± 0.5 °C with a stirring rate of 100 rpm. The concentration measurements were performed using UV detection at 326 nm.

The intrinsic dissolution rate (IDR) was calculated by dividing the dissolution rate by the exposure area of pellet (0.0754 cm²). The results are shown in table 2c below.

**Table 2c. Average (IDR) for compound 1 (free base), compound 1d (phosphate salt) and compound 1c (monohydrate form) in 0.01 M HCl at 100 rpm (wherein n represents the number of vessels).**

| **Compound** | **n** | **IDR (mg.min⁻¹.cm²)** |
|---|---|---|
| **1** (free base) | 2 | 0.8170 |
| **1d** (phosphate salt) | 3 | 2.5172 |
| **1c** (monohydrate form) | 2 | 1.1011 |

Compound **1d** (phosphate salt) showed a fast intrinsic dissolution rate in 0.01 M HCl compared to compounds **1** and **1c** (free base and hydrated forms, respectively). The absorbance values measured for the phosphate form after 5 min dissolution were over the UV detector limit. The pellets of compound **1d** (phosphate salt) were totally dissolved within 10 min. The pellets of compounds **1** and **1c** (free base and hydrated forms, respectively) showed similar intrinsic dissolution profile rates. The tablets started to break after 20 minutes dissolution.
Compound **1d** (phosphate salt) showed the highest IDR value (e.i. 2.52 mg.min⁻¹.cm²). The free base (compound **1**) and hydrated (compound **1c**) forms presented lower IDR with similar values (e.i. 0.82 and 1.10 mg.min⁻¹.cm⁻²).

Based on the above *in vitro* results, compound **1d**, i.e. the phosphate salt of compound **1**, constitutes an advantageous salt form of compound **1**, having a much higher solubility compared to that of the anhydrous free base form (compound **1**), and a higher intrinsic dissolution rate compared to that of both, compounds **1** and **1c** (the anhydrous free base and the monohydrate form, respectively). Additionally, compound **1d** constitutes a solid form of robust scalable manufacturability, allowing reproducible stoichiometry control in the salt formation, while the formation of compounds **1a** and **1c** (hydrochloride salt (.HCl) and the hydrate form (.H₂O) of compound **1**, respectively) does not allow reproducible stoichiometric control when scaled up. Finally, the melting point measured for compound **1d** (DSC, mp = 165.1°C) is higher than that measured for compound **1b**, i.e. the maleate salt form of compound **1**, (DSC, mp = 113.8 °C) which may provide higher resistance to potential salt dissociation phenomena upon mechanical manipulation and formulation during production and/or upon storage of the formulated product.

### Prophetic composition examples

"Active ingredient" as used throughout these examples relates to compound **1d**. Typical examples of recipes for the formulation of the invention are as follows:

### 1. Tablets

| | |
|---|---|
| Active ingredient | 5 to 50 mg |
| Di-calcium phosphate | 20 mg |
| Lactose | 30 mg |
| Talcum | 10 mg |
| Magnesium stearate | 5 mg |
| Potato starch | ad 200 mg |

### 2. Suspension

An aqueous suspension is prepared for oral administration so that each 1 milliliter contains 1 to 5 mg of one of the active compound, 50 mg of sodium carboxymethyl cellulose, 1 mg of sodium benzoate, 500 mg of sorbitol and water ad 1 ml.

### 3. Injectable

A parenteral composition is prepared by stirring 1.5 % by weight of active ingredient of the invention in 10% by volume propylene glycol in water.

### 4. Ointment

| | |
|---|---|
| Active ingredient | 5 to 1000 mg |
| Stearyl alcohol | 3 g |
| Lanoline | 5 g |
| White petroleum | 15 g |
| Water | ad 100 g |

## Claims

1. A phosphate salt of formula

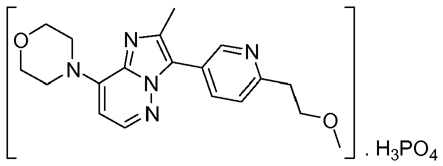

2. A pharmaceutical composition comprising:
(a) a therapeutically effective amount of the phosphate salt as defined in claim 1;
and
(b) at least one pharmaceutically acceptable carrier, diluent, vehicle or excipient.

3. The phosphate salt as defined in claim 1, for use as a medicament.

4. The phosphate salt, as defined in claim 1, or the pharmaceutical composition as defined in claim 2, for use in the treatment or prevention of a central nervous system disorder selected from the group of psychotic disorders, drug induced psychosis, anxiety disorders, movement disorders, mood disorders, neurodegenerative disorders, disorders or conditions comprising as a symptom a deficiency in attention and/or cognition, drug addiction disorders, and pain; or of a metabolic disorder.

5. The phosphate salt or the pharmaceutical composition for use according to claim 4, wherein the psychotic disorder is selected from the group consisting of schizophrenia, delusional disorder, schizoaffective disorder, schizophreniform disorder and substance-induced psychotic disorder.

6. The phosphate salt as defined in claim 1 in combination with an additional pharmaceutical agent for use in the treatment or prevention of a condition as cited in claim 4 or 5.

7. Use of the phosphate salt, as defined in claim 1, or the pharmaceutical composition as defined in claim 2, for the manufacture of a medicament for the treatment or prevention of a central nervous system disorder selected from the group of psychotic disorders, drug induced psychosis, anxiety disorders, movement disorders, mood disorders, neurodegenerative disorders, disorders or conditions comprising as a symptom a deficiency in attention and/or cognition, drug addiction disorders, and pain; or of a metabolic disorder.

8. Use according to claim 7, wherein the psychotic disorder is selected from the group consisting of schizophrenia, delusional disorder, schizoaffective disorder, schizophreniform disorder and substance-induced psychotic disorder.

9. A process for preparing the phosphate salt as defined in claim 1, comprising the step of reacting 3-[6-(2-methoxyethyl)-3-pyridinyl]-2-methyl-8-(4-morpholinyl)-imidazo[1,2-b]pyridazine with phosphoric acid in the presence of a suitable solvent.

10. The process according to claim 9, wherein the solvent is an alcohol or a mixture of two or more alcohols.

11. The process according to claim 9 or 10, wherein the solvent is selected from methanol, ethanol, propanol, isopropanol, butanol, sec-butanol, tert-butanol, and mixtures thereof.

12. A process for the preparation of the pharmaceutical composition as defined in claim 2, **characterized in that** a pharmaceutically acceptable carrier is intimately mixed with a therapeutically effective amount of the phosphate salt as defined in claim 1.

13. A product comprising
(a) the phosphate salt as defined in claim 1; and
(b) an additional pharmaceutical agent,
as a combined preparation for simultaneous, separate or sequential use in the treatment or prevention of a condition as cited in claim 4 or 5.

## Patentansprüche

1. Phosphatsalz der Formel

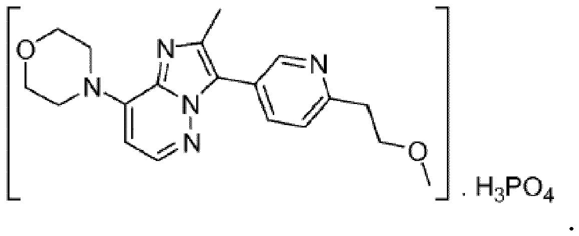

2. Pharmazeutische Zusammensetzung, umfassend:
(a) eine therapeutisch wirksame Menge des wie in Anspruch 1 definierten Phosphatsalzes und
(b) mindestens einen pharmazeutisch unbedenklichen Träger, ein pharmazeutisch unbedenkliches Verdünnungsmittel, ein pharmazeutisch unbedenkliches Vehikel oder einen pharmazeutisch unbedenklichen Exzipienten.

3. Phosphatsalz, definiert wie in Anspruch 1, zur Verwendung als Medikament.

4. Phosphatsalz, definiert wie in Anspruch 1, oder pharmazeutische Zusammensetzung, definiert wie in Anspruch 2, zur Verwendung bei der Behandlung oder Prävention einer Erkrankung des zentralen Nervensystems ausgewählt aus der Gruppe bestehend aus psychotischen Erkrankungen, drogen-/arzneimittelinduzierter Psychose, Angststörungen, Bewegungsstörungen, Stimmungsstörungen, neurodegenerativen Erkrankungen, Erkrankungen bzw. Leiden, die als ein Symptom ein Aufmerksamkeits- und/oder Kognitionsdefizit umfassen, mit Drogenabhängigkeit in Zusammenhang stehenden Erkrankungen und Schmerzen oder einer Stoffwechselstörung.

5. Phosphatsalz oder pharmazeutische Zusammensetzung zur Verwendung nach Anspruch 4, wobei die psychotische Erkrankung ausgewählt ist aus der Gruppe bestehend aus Schizophrenie, paranoidem Syndrom, schizoaffektiver Erkrankung, schizophreniformer Erkrankung und substanzinduzierter psychotischer Erkrankung.

6. Phosphatsalz, definiert wie in Anspruch 1, in Kombination mit einem weiteren pharmazeutischen Mittel zur Verwendung bei der Behandlung oder Prävention eines wie in Anspruch 4 oder 5 angeführten Leidens.

7. Verwendung des wie in Anspruch 1 definierten Phosphatsalzes oder der wie in Anspruch 2 definierten pharmazeutischen Zusammensetzung zur Herstellung eines Medikaments zur Behandlung oder Prävention einer Erkrankung des zentralen Nervensystems ausgewählt aus der Gruppe bestehend aus psychotischen Erkrankungen, drogen-/arzneimittelinduzierter Psychose, Angststörungen, Bewegungsstörungen, Stimmungsstörungen, neurodegenerativen Erkrankungen, Erkrankungen bzw. Leiden, die als ein Symptom ein Aufmerksamkeits- und/oder Kognitionsdefizit umfassen, mit Drogenabhängigkeit in Zusammenhang stehenden Erkrankungen und Schmerzen oder einer Stoffwechselstörung.

8. Verwendung nach Anspruch 7, wobei die psychotische Erkrankung ausgewählt ist aus der Gruppe bestehend aus Schizophrenie, paranoidem Syndrom, schizoaffektiver Erkrankung, schizophreniformer Erkrankung und substanzinduzierter psychotischer Erkrankung.

9. Verfahren zur Herstellung des wie in Anspruch 1 definierten Phosphatsalzes, bei dem man 3-[6-(2-Methoxyethyl)-3-pyridinyl]-2-methyl-8-(4-morpholinyl)imidazo[1,2-b]pyridazin in Gegenwart eines geeigneten Lösungsmittels mit Phosphorsäure umsetzt.

10. Verfahren nach Anspruch 9, wobei es sich bei dem Lösungsmittel um einen Alkohol oder eine Mischung von zwei oder mehr Alkoholen handelt.

11. Verfahren nach Anspruch 9 oder 10, wobei das Lösungsmittel aus Methanol, Ethanol, Propanol, Isopropanol, Butanol, sek.-Butanol, tert.-Butanol und Mischungen davon ausgewählt ist.

12. Verfahren zur Herstellung der wie in Anspruch 2 definierten pharmazeutischen Zusammensetzung, **dadurch gekennzeichnet, dass** man einen pharmazeutisch unbedenklichen Träger innig mit einer therapeutisch wirksamen Menge des wie in Anspruch 1 definierten Phosphatsalzes mischt.

13. Produkt, umfassend:
(a) das wie in Anspruch 1 definierte Phosphatsalz und
(b) ein zusätzliches pharmazeutisches Mittel, als Kombinationspräparat für die gleichzeitige, getrennte oder aufeinanderfolgende Anwendung bei der Behandlung oder Prävention eines wie in Anspruch 4 oder 5 angeführten Leidens.

## Revendications

1. Sel de phosphate de formule

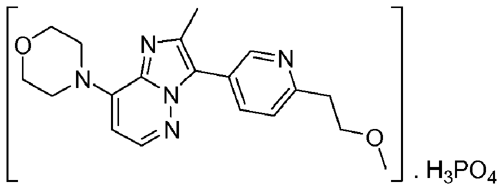

2. Composition pharmaceutique, comprenant :
(a) une quantité thérapeutiquement active du sel de phosphate tel que défini selon la revendication 1 ; et
(b) au moins un support, diluant, véhicule ou excipient pharmaceutiquement acceptable.

3. Sel de phosphate tel que défini selon la revendication 1, pour une utilisation comme médicament.

4. Sel de phosphate tel que défini selon la revendication 1, ou composition pharmaceutique telle que définie selon la revendication 2, pour une utilisation dans le traitement ou la prévention d'un trouble du système nerveux central choisi dans le groupe constitué par les troubles psychotiques, la psychose induite par les drogues, les troubles anxieux, les troubles du mouvement, les troubles de l'humeur, les troubles neurodégénératifs, les troubles ou conditions comprenant comme symptôme un déficit de l'attention et/ou de la cognition, les troubles de la toxicomanie, et la douleur ; ou d'un trouble métabolique.

5. Sel de phosphate ou composition pharmaceutique pour une utilisation selon la revendication 4, où le trouble psychotique est choisi dans le groupe constitué par la schizophrénie, le trouble délirant, le trouble schizo-affectif, le trouble schizophréniforme, et le trouble psychotique induit par les drogues.

6. Sel de phosphate tel que défini selon la revendication 1, en combinaison avec un agent pharmaceutique supplémentaire, pour une utilisation dans le traitement ou la prévention d'une condition telle que citée selon la revendication 4 ou 5.

7. Utilisation du sel de phosphate tel que défini selon la revendication 1, ou de la composition pharmaceutique telle que définie selon la revendication 2, pour l'élaboration d'un médicament destiné au traitement ou à la prévention d'un trouble du système nerveux central choisi dans le groupe constitué par les troubles psychotiques, la psychose induite par les drogues, les troubles anxieux, les troubles du mouvement, les troubles de l'humeur, les troubles neurodégénératifs, les troubles ou conditions comprenant comme symptôme un déficit de l'attention et/ou de la cognition, les troubles de la toxicomanie, et la douleur ; ou d'un trouble métabolique.

8. Utilisation selon la revendication 7, dans laquelle le trouble psychotique est choisi dans le groupe constitué par la schizophrénie, le trouble délirant, le trouble schizo-affectif, le trouble schizophréniforme, et le trouble psychotique induit par les drogues.

9. Procédé de préparation du sel de phosphate tel que défini selon la revendication 1, comprenant l'étape de réaction de 3-[6-(2-méthoxyéthyl)-3-pyridinyl]-2-méthyl-8-(4-morpholinyl)imidazo[1,2-b]pyridazine avec de l'acide phosphorique en présence d'un solvant convenable.

10. Procédé selon la revendication 9, dans lequel le solvant est un alcool ou un mélange de deux, ou plus, alcools.

11. Procédé selon la revendication 9 ou 10, dans lequel le solvant est choisi parmi le méthanol, l'éthanol, le propanol, l'isopropanol, le butanol, le sec-butanol, le tertio-butanol, et des mélanges de ceux-ci.

12. Procédé de préparation de la composition pharmaceutique telle que définie selon la revendication 2, **caractérisé en ce qu'**un support pharmaceutiquement acceptable est mélangé de manière intime avec une quantité thérapeutiquement efficace du sel de phosphate tel que défini selon la revendication 1.

13. Produit comprenant :
(a) le sel de phosphate tel que défini selon la revendication 1 ; et
(b) un agent pharmaceutique supplémentaire ;
comme préparation combinée pour une utilisation simultanée, séparée ou séquentielle dans le traitement ou la prévention d'une condition telle que citée selon la revendication 4 ou 5.
